# EUROPÄISCHE PATENTSCHRIFT

(11) **EP 0 905 204 B1**
(45) Veröffentlichungstag und Bekanntmachung des Hinweises auf die Patenterteilung: **20.11.2002**
(21) Anmeldenummer: 98117298.4
(22) Anmeldetag: 12.09.1998
(51) Int. Cl.: C09C 1/00, C11D 17/00, C09D 7/12, C09D 11/00, C08K 3/00, A61K 7/00

(54) **Oberflächenmodifizierte plättchenförmige Substrate**
Surface-modified platy substrates
Substrats en paillettes modifiées superficiellement

(30) Priorität: 26.09.1997 DE 19742551; 20.01.1998 DE 19801809
(43) Veröffentlichungstag der Anmeldung: 31.03.1999
(73) Patentinhaber: MERCK PATENT GmbH, 64293 Darmstadt (DE)
(72) Erfinder: Herget, Gerhard, Dr., 64372 Ober-Ramstadt (DE); Husseini, Brigitte, 64293 Darmstadt (DE)

(56) Entgegenhaltungen:
- EP-A- 0 523 357
- EP-A- 0 803 552

## Beschreibung

Die vorliegende Erfindung betrifft oberflächenmodifizierte Pigmente auf der Basis plättchenförmiger Substrate mit einem verbesserten Absetz- und Aufrührverhalten (gemäß Anspruch 1) sowie deren Herstellungsverfahren (gemäß Anspruch 6) und Verwendung.

Beschichtungsmassen, wie z.B. Lacke, Farben, Druckfarben, etc., enthaltend Pigmente auf der Basis plättchenförmiger Substrate sind insofern problematisch in der Handhabung, als die Pigmente infolge ihrer Größe und ihrer Dichte sich leicht absetzen und dann zu einem sehr festen Sedimentkuchen zusammenbacken können. Dieser Kuchen ist in der Regel nur schwer wieder aufrührbar. Dies gilt vor allem bei der Lagerung von Lacken, Farben und Druckfarben, sowie bei der Verarbeitung derselben. Weiterhin wird bei der Pigmentierung der Kunststoffe, Basislacksysteme, etc. vielfach eine Agglomeration der Pigmente beobachtet. Eine homogene Verteilung des Pigments in der jeweiligen Matrix ist nur schwer oder gar nicht zu erreichen.

So wurden unter anderem zahlreiche Methoden entwickelt, um das Problem der Einarbeitung und Handhabung von plättchenförmigen Pigmenten in Beschichtungsmassen zu lösen.

Aus der DE 36 27 329 und den EP's 0 306 056 und 0 268 918 ist bekannt, daß modifizierte plättchenförmige Substrate mit einer Polymerbeschichtung bzw. nach Behandlung mit Kupplungsreagenzien wie Organotitanaten, Organosilanen in Belegmassen ein verbessertes Absetz- und Aufrührverhalten zeigen.

Weiterhin kann das Wiederaufrühren erleichtert werden, indem den Beschichtungsmassen Additive zugesetzt werden, die entweder eine gezielte Flokkulation (Kartenhauseffekt), ein strukturviskoses und/oder thixotropes Verhalten, eine sterische Abstoßung und/oder eine elektrostatische Abstoßung der Pigmente bewirken.

Additive mit thixotropem Verhalten sind in der EP 0 198 519 und der DE OS 18 05 693 beschrieben. Aus der DE 39 22 178 ist bekannt, daß man durch Mischen einer Suspension eines plättchenförmigen Substrats mit sphärischen Teilchen, wie z.B. SiO₂, TiO₂ und ZrO₂, deagglomerierte und gut dispergierbare Pigmente erhält.

In der EP 0 523 357 werden Perlglanzpigmente mit Diharnstoffderivaten behandelt, während aus der EP 0 515 928 die Oberflächenmodifizierung von Pigmenten mit Polyacrylaten bekannt ist. Aus der EP 0 650 144 ist die Beschichtung von Perlglanzpigmenten mit SiO₂/Al₂O₃ und SiO₂/ZrO₂ bekannt.

Alle diese Zusätze können jedoch einen negativen Einfluß auf die Qualität der Beschichtung haben. Insbesondere die Brillanz und Koloristik bei Perlglanzpigmenten und die Gleichmäßigkeit der Beschichtung können beeinträchtigt werden. Diese Beeinträchtigungen sind im allgemeinen umso größer, je höher die Einsatzkonzentration der Additive ist.

Es bestand daher die Aufgabe oberflächenmodifizierte Pigmente auf der Basis plättchenförmiger Substrate zu finden, die bei Einarbeitung in einer Beschichtungsmasse die bei herkömmlich pigmentierten Beschichtungsmassen beobachteten Nachteile nicht oder nur in geringem Umfang zeigen.

Überraschenderweise wurde nun gefunden, daß Pigmente, welche auf plättchenförmigen Substraten basieren und mit einem Schichtsilikat vorzugsweise aus der Gruppe der Smectite mit einem Durchmesser von 0,1 - 25 nm [Teilchengröße im aktivierten (vollständig dispergiertem) Zustand] beschichtet sind, ein verbessertes Absetz- und Aufrührverhalten in Beschichtungsmassen zeigen.

Gegenstand der Erfindung sind daher oberflächenmodifizierte Pigmente, welche auf einem plättchenförmigen Substrat basieren, die zur Verbesserung des Absetz- und Aufrührverhaltens mit einem Schichtsilikat mit einem Durchmesser von 0,1 - 25 nm beschichtet sind.

Gegenstand der Erfindung ist auch ein Verfahren zur Herstellung oberflächenmodifizierter plättchenförmiger Substrate, dadurch gekennzeichnet, daß man in einem Mischgefäß plättchenförmige Substrate mit einem Schichtsilikat vorzugsweise aus der Gruppe der Smectite, trocken mischt.

Die einfache Mischung von Perlglanzpigment mit Schichtsilikat zeigt in mehr oder weniger unzulässiger Weise Agglomerationen und starken Glanzverlust. Überraschenderweise wurde nun gefunden, daß bei einer Vorbehandlung des Wirkstoffs (Dispergieren und Sprühtrocknen) ein Pulver erzeugt werden kann, das nur wenig Agglomerationsneigung und keinen Glanzverlust mehr zeigt.

Überraschend ist auch die Beobachtung, daß die beschichteten Substrate in ihren optischen Eigenschaften nicht nachhaltig beeinflußt werden. Die Einsatzkonzentration des natürlichen oder synthetischen Silikats kann 0,5 - 30 Gew.% bezogen auf das Pigment betragen, vorzugsweise liegt sie jedoch bei 0,5 - 20 Gew.%, insbesondere bei 0,5 - 5 Gew.%. Die erfindungsgemäßen Pigmente zeichnen sich weiterhin durch eine gute Kompatibilität mit den übrigen Komponenten von Beschichtungssystemen sowie durch ihre gute Verarbeitbarkeit, die hohe Staubfreiheit und Stabilität aus. Bei der Einarbeitbarkeit der Pigmente in Formulierungen ist ein verzögertes Absetzverhalten und ein verbessertes Wiederaufrührverhalten eines aufgetretenen Bodensatzes zu beobachten. Aufgrund des sehr guten skin-feelings sind die erfindungsgemäßen Pigmente auch sehr gut für kosmetische Formulierungen geeignet.

Alle bekannten plättchenförmigen Metalle, Metalloxide, Glimmerpigmente und sonstigen plättchenförmigen Substrate können nach dem erfindungsgemäßen Verfahren belegt werden.

Beispiele hierfür sind natürlicher und synthetischer Glimmer, Talkum, Kaolin, SiO₂-Flakes, TiO₂-Flakes, Aluminium-Flakes, oder andere vergleichbare Mineralien, plättchenförmiges Eisenoxid, LCPs, holographische Pigmente und Wismutoxichlorid.

Da bei dem Verfahren keine hohen Scherkräfte benötigt werden, ist das Verfahren auch hervorragend zur Beschichtung von Perlglanzpigmenten geeignet.

Es können alle üblichen Perlglanzpigmente verwendet werden, z.B. Glimmerbeschichtungen mit farbigen oder farblosen Metalloxiden, wie TiO₂, Fe₂O₃, SnO₂, Cr₂O₃, ZnO und anderen Metalloxiden, allein oder in Mischung in einer einheitlichen Schicht oder in aufeinanderfolgenden Schichten. Diese Pigmente sind z.B. aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 35 017 bekannt und im Handel erhältlich, z.B. unter der Marke Iriodin® von der Firma Merck KGaA, Darmstadt. Weiterhin können auch leitfähige Pigmente, z.B. wie sie aus der EP 0 373 575 bekannt sind und im Handel unter der Marke Minatec® von der Firma Merck KGaA, Darmstadt, vertrieben werden, modifiziert werden.

Geeignete Schichtsilikate sind vor allem solche aus der Gruppe der Smektitreihe, wie z.B. Montmorillonit-/Beidellit-Reihe. Die genannten Smectite zeichnen sich insbesondere durch ihr ausgeprägtes Quellverhalten aus. Im Handel werden diese Produkte z.B. unter der Marke Laponite, ein synthetisches Natrium-Magnesium-Lithium-Silikat, welches dem Hectorit ähnelt, von der Fa. Laporte UK oder von der Fa. Südchemie, BRD unter den Namen Optigel CG, ein Bentonit, bzw. Tixogel PE, ein organophiles hydrophobiertes Smectit, vertrieben. Es kommen aber auch glimmerartige Silikate der Vermiculit- und der Illitreihe sowie die Glimmer selbst ( Muscovit, Phlogopit, Biotit) in Frage. Entscheidend ist die plättchenförmige Beschaffenheit der Partikel und ihre Aufschließbarkeit im Sprühtrockenprozeß.

Bei der Oberflächenmodifizierung können auch Gemische der genannten Schichtsilikate sowie modifizierte Schichtsilikate eingesetzt werden.

Vorzugsweise werden die Schichtsilikate in einer aktivierten Form eingesetzt, d.h., die Silikate werden in Wasser dispergiert, in ein Sol überführt und anschließend sprühgetrocknet.

Der Einsatz eines aktivierten Schichtsilikats erleichtert insofern die Oberflächenmodifizierung von Pigmenten, da das Anteigen des Pigments entfallen kann und kein Lösungsmittel benötigt wird.

Bei der Solbildung können der wäßrigen Dispersion auch übliche Additive, wie z.B. Dispergierhilfsmittel, wie z.B. Polyphosphate, insbesondere Natriumpyrophosphat oder Natriumhexametaphosphat, zugesetzt werden. Die Gesamtkonzentration aller Additive bezogen auf das Schichtsilikat sollte jedoch 10 Gew.% nicht übersteigen. Vorzugsweise sollte der Gehalt an Additiven zwischen 0,1 und 5 Gew.%, insbesondere 0,5 - 2 Gew.%, liegen.

Das erfindungsgemäße Verfahren ist somit einfach und läßt sich leicht handhaben. Die Herstellung der Präparation geschieht durch trockenes Vermischen der genannten Wirksubstanzen mit dem Pigment, z.B. in einem Taumel-, Flügelrad-, Schaufel- oder Fluidmischer, wobei auf Grund der relativ hohen Bruchanfälligkeit der Substrate langsam laufende Mischer bevorzugt werden. Da keine hohen Scherkräfte bei der Pigmentpräparation notwendig sind, ist das erfindungsgemäße Verfahren auch für Perlglanzpigmente hervorragend geeignet.

Gegebenenfalls kann dem Gemisch aus Pigment und Modifiziermittel noch ein Feuchthaltemittel zugesetzt werden.

Die erfindungsgemäßen Pigmente können ebenfalls zur Herstellung von Pigmentpräparationen eingesetzt werden. Hierfür werden die Pigmente mit 0,5 - 30 Gew.% Wasser oder einem organischen Lösungsmittel oder eines Lösungsmittelgemisches angeteigt. Derartige Pigmentpräparationen enthalten mindestens 80 Gew.% der erfindungsgemäßen Pigmente. Weiterhin kann die wäßrige bzw. lösemittelhaltige Mischung noch bis zu 30 Gew.% Bindemittel bzw. Harz enthalten. Die Harze sind vorzugsweise ausgewählt aus der Reihe der im Druckbereich eingesetzten Harze. Der Pigmentgehalt dieser Mischungen beträgt dann mindestens 40 Gew.%. Die Pigmentpräparation kann zusätzliche Komponenten, wie z.B. Entschäumer, Netzmittel, Antiabsetzmittel, Verlaufsmittel, Sikkative, Thixotropiemittel enthalten. Weiterhin kann die Pigmentpräparation stranggepreßt, gegebenenfalls getrocknet und pelletiert werden. Im getrockneten Zustand beträgt der Pigmentgehalt mindestens 70 Gew.%. Die Pigmentpräparationen und daraus hergestellten Trockenpräparate können in alle bekannten Anwendungsmedien eingearbeitet werden, insbesondere in Lacken, Farben und Druckfarben.

Gegenstand der Erfindung sind ebenfalls Pigmentpräparationen mit den erfindungsgemäßen Pigmenten.

Die erfindungsgemäßen Pigmente sind mit einer Vielzahl von Farbsystemen kompatibel vorzugsweise aus dem Bereich der Lacke, Farben und Druckfarben.

Gegenstand der Erfindung ist somit auch die Verwendung der oberflächenmodifizierten Pigmente in Formulierungen wie Farben, Druckfarben, Lacken, Kunststoffen und zur Kosmetikpräparation.

Die nachfolgenden Beispiele sollen die Erfindung erläutern, ohne sie zu begrenzen.

### Beispiele

### Beispiel 1

Zu 1800 g VE-Wasser werden bei 40 °C 200 g Laponite RDS (Produkt der Fa. Laporte, UK) unter Rühren bei 500 Upm zugegeben und bei 700 Upm 1 Stunde weitergerührt. Es entsteht ein Sol mit einer Viskosität von 12 Sek. 4 mm DIN-Becher.

Das Sol wird in einem Sprühtrockner Atomizer Minor der Fa. Niro mit einem theoretischen Durchsatz von 5 - 7 l/Stunde bei 95 - 100 °C und 4 bar getrocknet.

### Beispiel 2

Zu 1900 g einer 10 %igen Natriumpyrophosphat-Lösung, die auf 40 °C erwärmt wird, werden unter Rühren an einem Dispermat AE der Fa. Getzmann mit Zahnscheibe Durchmesser 6 cm, 100 g Laponite RD (Fa. Laporte, UK) gegeben und 2 x 5 Min. bei 2000 Upm homogenisiert. Anschließend wird mit 900 g VE-Wasser in einen 3 l-Stutzen überführt und nochmals 10 Min. bei 800 Upm gerührt. Das Sol wird in einem Sprühtrockner Atomizer Minor der Fa. Niro bei 95 - 110 °C und 4 bar getrocknet.

### Beispiel 3

Zu 1900 g einer 10 %igen Natriumpyrophosphat-Lösung, die auf 40 °C erwärmt wird, wird unter Rühren an einem Dispermat AE der Fa. Getzmann mit einer Zahnscheibe, Durchmesser 6 cm, 1000 g Optigel CG (Fa. Südchemie, BRD) eingetragen und 2 x 5 Min. bei 2000 Upm homogenisiert. Anschließend wird mit 900 g VE-Wasser in einen 3 l-Stutzen überführt und nochmals 10 Min. bei 800 Upm gerührt. Das Sol wird in einem Sprühtrockner Niro-Atomizer bei 95 - 110 °C und 4 bar getrocknet.

### Beispiel 4

250 g Iriodin 123 Hellglanzsatin (TiO₂/Glimmer-Pigment der Teilchengröße 10 - 60 µm der Fa. Merck KGaA, BRD) werden mit 3,8 g sprühgetrocknetem Laponite RDS aus Versuch 1 trocken vermischt. Die Mischzeit beträgt 15 Minuten. Es entsteht ein homogenes Material, das in dieser Form für die weiteren Versuche eingesetzt wird.

### Beispiel 5

Das Pigment aus Versuch 4 wird gegen das Ausgangsmaterial Iriodin 123 in einer Lackkarte verglichen (Pigmentkonzentration 1,7 Gew.%, Nitrocellulose/Polyacrylat-Bindemittel). Es gibt visuell keine Unterschiede.

Die Messung der Lab-Werte an Hunterlab-Spektralgoniometer ergibt keine Abweichungen von der Meßtoleranz.

### Beispiel 6

39 g Pigment aus Versuch 4 werden in 91 g eines Bindemittels Synthacryl SW175 (40%ige Acrylharzlösung in H₂O/Isopropanol 2:1) der Fa. Hoechst eingearbeitet und mit Isopropanol auf eine Auslaufzeit von 21 +/- 1 Sekunden gestellt. Die Pigmentgehalt beträgt 22,3 Gew.%.

### Beispiel 7

39 g Iriodin 123 Hellglanzsatin werden in 91 g Bindemittel Synthacryl SW175 der Fa. Hoechst eingearbeitet und mit Isopropanol auf eine Auslaufzeit von 21 +/- 1 Sekunden gestellt. Der Pigmentgehalt beträgt 24,2 Gew.%.

### Beispiel 8

Je 50 ml der Farben aus den Versuchen 6 und 7 werden in Meßzylindern 30 Tage auf ihr Absetzverhalten untersucht. Nach 30 Tagen beträgt das Sediment des modifizierten Pigments 30 ml und ist sehr leicht wieder aufrührbar, während das Sediment der Referenz 20 ml beträgt und nur mittelgut aufrührbar ist. Ein Dorn durchdringt das Sediment des modifizierten Pigmentes ganz, während die Referenz nur bis zu 65 % Eindringtiefe zeigt.

### Beispiel 9

39 g Pigment aus Versuch 4 werden in 91 g eines Bindemittels GS 95 MB011TW (Nitrocellulosebasis) der Fa. Gebrüder Schmidt, Frankfurt, eingearbeitet und mit 1-Ethoxy-2-propanol auf eine Auslaufzeit von 21 Sekunden 4 mm DIN-Becher gestellt. Die Pigmentkonzentration beträgt 21,6 Gew.%.

### Beispiel 10

39 g Iriodin 123 werden in 91 g eines Bindemittels GS 95 MB011TW der Fa. Gebrüder Schmidt, Frankfurt, eingearbeitet und mit 1-Ethoxy-2-propanol auf eine Auslaufzeit von 21 Sekunden 4 mm DIN-Becher gestellt. Die Pigmentkonzentration beträgt 23,6 Gew.%.

### Beispiel 11

Je 50 ml der Farben aus den Versuchen 9 und 10 werden in Meßzylindern 30 Tage auf ihr Absetzverhalten untersucht. Nach 30 Tagen betragen alle Sedimente 25 ml. Die Aufrührbarkeit ist allgemein schwer, jedoch ist das modifizerte Pigment feststellbar leichter aufrührbar als die Referenz.

## Patentansprüche

1. Oberflächenmodifizierte Pigmente auf Basis plättchenförmiger Substrate, **dadurch gekennzeichnet, daß** sie zur Verbesserung des Absetz- und Aufrührverhaltens mit einem Schichtsilikat oder Schichtsilikatgemisch mit einem Durchmesser von 0,1 - 25 nm beschichtet sind.

2. Oberflächenmodifizierte Pigmente nach Anspruch 1, **dadurch gekennzeichnet, daß** der Gehalt an Schichtsilikat bezogen auf das Gesamtpigment 0,2 - 30 Gew. % beträgt.

3. Oberflächenmodifizierte Pigmente nach Anspruch 1 oder 2, **dadurch gekennzeichnet, daß** das Schichtsilikat aus der Gruppe der Smectite ist.

4. Oberflächenmodifiziertes Pigment nach einem der Ansprüche 1 bis 3, **dadurch gekennzeichnet, daß** das plättchenförmige Substrat ausgewählt ist aus der Gruppe der Metalle, Metalloxide, Glimmerpigmente, Liquid Crystal Polymers (LCPs), holographischen Pigmente, Wismutoxichlorid, Perlglanzpigmente und leitfähigen Pigmente.

5. Oberflächenmodifiziertes Pigment nach Anspruch 4, **dadurch gekennzeichnet, daß** das Perlglanzpigment ein mit farbigen oder farblosen Metalloxiden in einer einheitlichen Schicht oder in aufeinanderfolgenden Schichten beschichtetes Glimmerplättchen ist.

6. Verfahren zur Herstellung oberflächenmodifizierter plättchenförmiger Substrate nach Anspruch 1, **dadurch gekennzeichnet, daß** man in einem Mischgefäß plättchenförmige Substrate mit dem Schichtsilikat trocken mischt.

7. Verfahren nach Anspruch 6, **dadurch gekennzeichnet, daß** das Schichtsilikat in aktivierter Form eingesetzt wird, indem das Silikat in das Sol überführt wird, dem gegebenenfalls noch Additive zugesetzt werden können und das Sol zuletzt sprühgetrocknet wird.

8. Verfahren nach Anspruch 7, **dadurch gekennzeichnet, daß** dem Sol vor der Sprühtrocknung ein Dispergiermittel zugesetzt wird.

9. Verwendung der oberflächenmodifizierten Pigmente nach Anspruch 1 in Formulierungen wie Farben, Lacken, Druckfarben, Kunststoffen und zur Kosmetikpräparation.

10. Pigmentpräparationen, **dadurch gekennzeichnet, daß** sie mindestens 80 % der Pigmente nach Anspruch 1 sowie 0,5 - 30 Gew. % an Wasser oder eines organischen Lösungsmittels oder eines Lösungsmittelgemisches enthalten.

11. Pigmentpräparationen, **dadurch gekennzeichnet, daß** sie mindestens 70 Gew. % an oberflächenmodifizierten Pigmenten gemäß Anspruch 1 und bis zu 30 Gew.% % Bindemittel bzw. Harz enthalten.

12. Verwendung der Pigmentpräparation nach Anspruch 10 oder 11 zur Herstellung von Trockenpräparaten, **dadurch gekennzeichnet, daß** die Präparation stranggepresst, pelletiert oder granuliert und anschließend gegebenenfalls getrocknet wird.

13. Verwendung der Pigmentpräparation nach Anspruch 10 in Formulierungen wie Farben, Lacken, Druckfarben und Kunststoffen.

## Claims

1. Surface-modified pigments based on platelet-shaped substrates, **characterised in that**, in order to improve the settling and stirring behaviour, they are coated with a phyllosilicate or phyllosilicate mixture having a diameter of 0.1 - 25 nm.

2. Surface-modified pigments according to Claim 1, **characterised in that** the phyllosilicate content, based on the pigment as a whole, is 0.2 - 30% by weight.

3. Surface-modified pigments according to Claim 1 or 2, **characterised in that** the phyllosilicate is selected from the group consisting of the smectites.

4. Surface-modified pigments according to one of Claims 1 to 3, **characterised in that** the platelet-shaped substrate is selected from the group consisting of metals, metal oxides, mica pigments, liquid crystal polymers (LCPs), holographic pigments, bismuth oxychloride, pearlescent pigments and conductive pigments.

5. Surface-modified pigment according to Claim 4, **characterised in that** the pearlescent pigment is a mica platelet coated with coloured or colourless metal oxides in a uniform layer or in successive layers.

6. Process for the production of surface-modified platelet-shaped substrates according to Claim 1, **characterised in that** platelet-shaped substrates are dry-mixed with the phyllosilicate in a mixing vessel.

7. Process according to Claim 6, **characterised in that** the phyllosilicate is employed in activated form by converting the silicate into the sol, to which, if desired, additives may be added, and finally spray-drying the sol.

8. Process according to Claim 7, **characterised in that** a dispersant is added to the sol before the spray drying.

9. Use of the surface-modified pigments according to Claim 1 in formulations, such as paints, coatings, printing inks, plastics, and for cosmetic preparations.

10. Pigment preparations, **characterised in that** they comprise at least 80% of the pigments according to Claim 1 and 0.5 - 30% by weight of water or an organic solvent or a solvent mixture.

11. Pigment preparations, **characterised in that** they comprise at least 70% by weight of surface-modified pigments according to Claim 1 and up to 30% by weight of binder or resin.

12. Use of the pigment preparation according to Claim 10 or 11 for the preparation of dry preparations, **characterised in that** the preparation is extruded, pelletised or granulated and subsequently, if necessary, dried.

13. Use of the pigment preparation according to Claim 10 in formulations, such as paints, coatings, printing inks and plastics.

## Revendications

1. Pigments modifiés en surface à base de substrats sous forme lamellaire, **caractérisés en ce que** pour améliorer les comportements à la sédimentation et à l'agitation on les revêt de phyllosilicate ou d'un mélange de phyllosilicate ayant un diamètre compris entre 0,1 et 25 nm.

2. Pigments modifiés en surface selon la revendication 1, **caractérisés en ce que** la teneur en phyllosilicate rapportée à la totalité des pigments est comprise entre 0,2 et 30 % en poids.

3. Pigments modifiés en surface selon la revendication 1 ou 2, **caractérisés en ce que** le phyllosilicate provient du groupe des smectites.

4. Pigment modifié en surface selon l'une des revendications 1 à 3, **caractérisé en ce que** le substrat sous forme lamellaire est choisi dans le groupe constitué par les métaux, les oxydes métalliques, les pigments de mica, les polymères cristaux liquides (PCL), les pigments holographiques, l'oxychlorure de bismuth, les pigments nacrés et les pigments conducteurs.

5. Pigment modifié en surface selon la revendication 4, **caractérisé en ce que** le pigment nacré est une lamelle de mica revêtue d'oxydes métalliques colorés ou incolores en une couche homogène ou en couches successives.

6. Procédé pour la préparation de substrats sous forme lamellaire modifiés en surface selon la revendication 1, **caractérisé en ce que** l'on mélange à sec dans un mélangeur les substrats sous forme lamellaire et le phyllosilicate.

7. Procédé selon la revendication 6, **caractérisé en ce que** l'on utilise le phyllosilicate sous forme activée, en transformant le silicate en sol auquel peuvent être éventuellement encore ajoutés des additifs et en séchant finalement le sol par pulvérisation.

8. Procédé selon la revendication 7, **caractérisé en ce que** l'on ajoute au sol avant le séchage par pulvérisation un agent dispersant.

9. Utilisation des pigments modifiés en surface selon la revendication 1 dans des formulations telles que des couleurs, vernis, encres d'imprimerie, matières plastiques et pour les préparations cosmétiques.

10. Préparations pigmentaires, **caractérisées en ce qu'**elles contiennent au moins 80 % de pigments selon la revendication 1, ainsi que 0,5 à 30 % en poids d'eau ou d'un solvant organique ou d'un mélange de solvants.

11. Préparations pigmentaires, **caractérisées en ce qu'**elles contiennent au moins 70 % en poids de pigments modifiés en surface selon la revendication 1 et jusqu'à 30 % en poids de liant ou de résine.

12. Utilisation de la préparation pigmentaire selon la revendication 10 ou 11, pour la réalisation de préparations sèches, **caractérisée en ce que** la préparation est extrudée, mise sous forme de pastilles ou de granulés, puis éventuellement séchée.

13. Utilisation de la préparation pigmentaire selon la revendication 10 dans des formulations telles que des couleurs, vernis, encres d'imprimerie et matières plastiques.
